# EUROPÄISCHE PATENTSCHRIFT

(11) **EP 3 305 168 B1**
(45) Veröffentlichungstag und Bekanntmachung des Hinweises auf die Patenterteilung: **22.05.2019**
(21) Anmeldenummer: 17189190.6
(22) Anmeldetag: 04.09.2017
(51) Int. Cl.: A61B 1/00, A61B 1/07, A61B 1/06

(54) **ENDOSKOP UND VERFAHREN ZUM FIXIEREN EINES BÜNDELS VON LICHTLEITERN IN EINEM SCHAFT EINES ENDOSKOPS**
ENDOSCOPE AND METHOD FOR FIXING A BUNDLE OF OPTICAL CONDUCTORS IN A SHAFT OF AN ENDOSCOPE
ENDOSCOPE ET PROCÉDÉ DE FIXATION D'UN FAISCEAU DE FIBRES OPTIQUES DANS UN ARBRE D'UN ENDOSCOPE

(30) Priorität: 26.09.2016 DE 102016118102
(43) Veröffentlichungstag der Anmeldung: 11.04.2018
(73) Patentinhaber: Henke-Sass, Wolf GmbH, 78532 Tuttlingen (DE)
(72) Erfinder: MATTES, Andreas, 78589 Dürbheim (DE); REHE, Oliver, 78573 Wurmlingen (DE)
(74) Vertreter: Patentanwälte Geyer, Fehners & Partner mbB

(56) Entgegenhaltungen:
- DE-A1-102004 023 024
- DE-A1-102013 112 282
- DE-A1-102014 111 069
- DE-B3-102007 044 663
- US-A- 4 850 342

## Beschreibung

Die Erfindung betrifft ein Endoskop mit den Merkmalen des Oberbegriffs des Anspruches 1.

Ferner betrifft die Erfindung ein Verfahren zum Fixieren eines Bündels von Lichtleitern in einem Schaft eines Endoskops durch Bereitstellen eines Mantelrohrs, welches sich entlang einer Längsachse erstreckt, und durch Bereitstellen eines Innenrohrs.

Endoskope werden z.B. verwendet, um von außen nicht sichtbare Körperöffnungen optisch zu untersuchen. Dazu weisen aus dem Stand der Technik bekannte Endoskope einen Schaft und ein Hauptteil auf. An einem distalen Ende des Schafts, also ein dem Hauptteil abgewandten Ende des Schafts, ist ein Objektiv vorgesehen und an dem Hauptteil ein Okular. Das distale Ende des Schafts wird in die Körperöffnung eingeführt und das Innere der Körperöffnung wird über das Objektiv und das Okular abgebildet. Um die Körperöffnung auch seitlich einer Längsachse des Schafts zu beobachten, ist es bei bekannten Endoskopen vorgesehen, eine optische Achse des Objektivs schräg zu der Längsachse des Schafts anzuordnen; die optische Achse des Objektivs und die Längsachse des Schaftes schließen demnach einen Winkel ein.

Ferner ist es bei Endoskopen bekannt, Lichtleiter durch den Schaft zu führen, mittels welchen das Körperinnere beleuchtet werden kann. Um die Lichtleiter an dem distalen Ende des Schafts hinsichtlich der Orientierung des Objektivs auszurichten, ist es bekannt, einen Keil am distalen Ende des Schafts zwischen die Lichtleiter und einem Rohr des Schaftes bei der Montage des Endoskops einzuführen, so dass die Lichtleiter annähernd parallel zu der optischen Achse an dem distalen Ende ausgerichtet werden.

Die DE 10 2014 111 069 A1, die DE 10 2013 112 282 A1 sowie die DE 10 2004 023 024 A1 beschreiben jeweils ein Endoskop mit einem Hauptteil und einem mit dem Hauptteil verbundenen Schaft, der sich entlang einer Längsachse erstreckt, wobei in dem Schaft ein Bündel von Lichtleitern angeordnet ist, das am distalen Ende des Innenrohres so angeordnet ist, dass die Lichtleiter nicht-parallel zur Längsachse verlaufen. Bei der DE 10 2004 023 024 A1 wird dabei das Faserbündel zwischen Anlageflächen eines Innen- und eines Außenrohres ausgerichtet und in mehrere Teilbündel geteilt.

Aufgabe der Erfindung ist es, ein Endoskop und ein Verfahren zum Fixieren eines Bündels von Lichtleitern in einem Schaft eines Endoskops bereitzustellen, das besonders zuverlässig und schnell hergestellt bzw. durchgeführt werden kann.

Die Erfindung ist im Anspruch 1 und im Anspruch 10 definiert. Die abhängigen Ansprüche beschreiben bevorzugte Ausführungsformen der Erfindung.

Die Erfindung betrifft ein Endoskop, welches ein Hauptteil und einen mit dem Hauptteil verbundenen Schaft aufweist, welcher sich entlang einer Längsachse erstreckt. Der Schaft umfasst ein Mantelrohr, ein in dem Mantelrohr angeordnetes Innenrohr zur Aufnahme eines Objektivs und ein Bündel von Lichtleitern, die in dem Schaft zwischen dem Mantelrohr und dem Innenrohr entlang der Längsachse verlaufen. Das Innenrohr weist an einem von dem Hauptteil abgewandten, distalen Ende einen Innenrohrkopf auf, welcher einen ersten Anlageabschnitt und einen zweiten Anlageabschnitt, der mit dem ersten Anlageabschnitt durch ein Verbindungselement verbunden ist, umfasst. Der erste Anlageabschnitt weist eine erste Anlagefläche, an dem das Bündel von Lichtleitern anliegt, und der zweite Anlageabschnitt eine zweite Anlagefläche, an dem das Bündel von Lichtleitern anliegt und welche der ersten Anlagefläche wenigstens bereichsweise gegenüberliegt, auf. Zur Ausrichtung des Bündels von Lichtleitern aus der Längsachse an dem distalen Ende verlaufen ein Bereich der ersten Anlagefläche und/oder ein Bereich der zweiten Anlagefläche nicht-parallel zu der Längsachse.

Vorteil des Endoskops ist, dass die Anordnung des Bündels von Lichtleitern an dem distalen Ende besonders einfach und zuverlässig dadurch gelingt, dass das Bündel von Lichtleitern zwischen den Bereichen der ersten und zweiten Anlagefläche, die sich gegenüberliegen, angeordnet wird. Dadurch, dass sich die entsprechenden Bereiche der ersten und zweiten Anlagefläche gegenüberliegen, kann eine provisorische Fixierung der relativen Lage der Lichtleiter zueinander erreicht werden. Das Einführen des Bündels von Lichtleitern zusammen mit dem Innenrohr in das Mantelrohr des Schafts kann dadurch erleichtert, da das Verrutschen der Lichtleiter zueinander erschwert ist. Gleichzeitig ist das Bündel von Lichtleitern an dem distalen Ende hinsichtlich der Orientierung des Objektivs ausgerichtet, da die erste Anlagefläche und/oder die zweite Anlagefläche gegenüber der Längsachse geneigt sind. Dies bedeutet, dass durch das Vorsehen des ersten Anlageabschnitts und des zweiten Anlageabschnitts das Einführen des Bündels von Lichtleitern vereinfacht werden kann und gleichzeitig die Ausrichtung des Bündels von Lichtleitern erreicht wird. Insbesondere durch die provisorische Anordnung des Bündels von Lichtleitern zwischen der ersten Anlagefläche und der zweiten Anlagefläche ist ein präzises Ausrichten des Bündels von Lichtleitern an dem distalen Ende möglich. Das nachträgliche Einführen eines Keils, wie es aus dem Stand der Technik bekannt ist, führte oft zu Verschiebungen in dem Bündel von Lichtleitern, so dass diese neu angeordnet werden müssen, während oder nachdem der Keil eingeführt wurde. Dies bedeutet, dass im Stand der Technik das Bündel von Lichtleitern zum Einführen in das Mantelrohr angeordnet wurde, dann der Keil eingebracht wurde und während oder anschließend oftmals das Bündel von Lichtleitern neu angeordnet werden muss. Bei dem Endoskop gemäß der Erfindung ist die Positionierung der Lichtleiter nur einmal notwendig, wobei durch das Bereitstellen von zwei Anlageflächen, die sich gegenüberliegen, die Anordnung deutlich vereinfacht werden kann, da das Bündel von Lichtleitern zwischen der ersten Anlagefläche und der zweiten Anlagefläche eingeklemmt werden kann.

Das Verbindungselement ist als Steg ausgebildet, wobei die erste Anlagefläche und die zweite Anlagefläche von dem Steg vorstehen. Der erste Anlageabschnitt, das Verbindungselement und der zweite Anlageabschnitt bilden in Draufsicht auf das distale Ende eine Doppel-T-Form. Der erste Anlageabschnitt und/oder der zweite Anlageabschnitt können/kann freie Enden aufweisen. Insbesondere können die beiden Anlageabschnitte (bzw. die beiden Anlageflächen) zusammen mit dem Verbindungselement in Draufsicht auf das distale Ende einen oder mehrere seitlich offene Aufnahmebereiche für die Lichtleiter bilden. Der Steg stellt eine besonders einfache Ausführungsform eines Verbindungselements dar. Es ist auch möglich, dass das Verbindungselement zwei oder mehr Stege umfasst.

Das Endoskop dient vorzugsweise zur Untersuchung einer Körperöffnung und weist optional abgesehen von dem distalen Ende einen Aufbau auf, wie er aus dem Stand der Technik bekannt ist. Das Hauptteil kann mittels Fügen mit dem Schaft verbunden sein, insbesondere Schweißen, Hartlöten oder Kleben. An dem Hauptteil kann ein Okular vorgesehen sein, welches zusammen mit einem an dem distalen Ende vorgesehenen Objektiv sowie optional mit in dem Schaft und/oder in dem Hauptteil angeordneten Linsen eine Abbildungsoptik bilden, mittels welcher das Innere einer Körperöffnung abgebildet werden kann. Das Okular ist an einem proximalen Ende des Endoskops vorgesehen.

An dem Hauptteil kann ferner ein optischer Anschluss vorgesehen sein, von welchem sich das Bündel von Lichtleitern bis zu dem distalen Ende erstreckt und mittels welchem Strahlung in das Bündel von Lichtleitern eingekoppelt werden kann. Dazu wird beispielsweise der optische Anschluss mit einer Lichtquelle, wie beispielsweise einer LED-Lichtquelle oder einer Halogen-Lichtquelle, verbunden.

Der Schaft erstreckt sich entlang der Längsachse, wobei sich auch das Innenohr, das Bündel von Lichtleitern und das Mantelrohr entlang der Längsachse erstrecken; dies gilt insbesondere nicht für einen Bereich an dem distalen Ende des Endoskops. Das Mantelohr stellt die Außenfläche des Schafts dar und in dessen Innenraum sind das Bündel von Lichtleitern und das Innenrohr angeordnet. In dem Innenrohr können Linsen, Stablinsen oder andere optische Elemente der Abbildungsoptik angeordnet sein. Der Lichtleiter kann eine optische Faser aus einem Polymer oder ähnlichen Materialien umfassen. Der Lichtleiter kann so ausgebildet sein, wie es aus dem Stand der Technik bekannt ist. Das Bündel umfasst insbesondere zwei oder mehr Lichtleiter, die an dem distalen Ende enden und dort das an dem optischen Anschluss eingekoppelte Licht abgeben, so dass an dem distalen Ende eine Vielzahl von Lichtquellen entsteht. Das Mantelrohr und/oder das Innenrohr sowie der Innenrohrkopf können aus einem metallischen Material, insbesondere Edelstahl, hergestellt sein.

Der Innenrohrkopf kann einstückig mit dem Innenrohr ausgebildet sein, jedoch ist es bevorzugt, dass das Innenrohr und der Innenrohrkopf zweiteilig ausgebildet sind. Optional ist der Innenrohrkopf mittels eines Fügeprozesses, wie beispielsweise Hartlöten, Kleben oder Schweißen, mit dem Innenrohr verbunden. Der Innenrohrkopf selbst kann als einstückige Einheit ausgebildet sein. Der Innenrohrkopf dient optional zur Fixierung des Objektivs und trägt zur Anordnung des Bündels von Lichtleitern bei. Um das Bündel von Lichtleitern an dem distalen Ende zu fixieren, können der Innenrohrkopf und das Bündel von Lichtleitern sowie optional das Mantelrohr mit Klebstoff verbunden sein.

Das Innenrohr, vorzugsweise der Innenrohrkopf, weiter vorzugsweise der erste Anlageabschnitt, weist optional eine Objektivaufnahme auf, in welchem das Objektiv angeordnet ist. Beispielsweise weist die Objektivaufnahme ein Gewinde auf, mittels welchem das Objektiv eingeschraubt werden kann. Eine optische Achse des Objektivs kann sich von dem Objektiv in Richtung des distalen Endes zu dem abzubildenden Objekt vor dem distalen Ende des Objektivs erstrecken. Die optische Achse ist insbesondere derart angeordnet, dass diese einen Winkel mit der Längsachse einschließt, wodurch ein Endoskop mit einem Blickwinkel von ungleich 0° bezogen auf die Längsachse bereitgestellt werden kann. Der Blickwinkel kann in einem Bereich von 5° bis 80°, bevorzugt von 15° bis 70° und weiter bevorzugt von 25° bis 60° liegen. Es sind auch Werte von 30° bis 45° oder 50° möglich. Ein Öffnungswinkel eines abbildbaren Bereichs kann z.B. ±10°, ±15° oder ±20° bezogen auf die optische Achse sein. Der Blickwinkel und der Öffnungswinkel legen das Sichtfeld des Endoskops fest. Die optische Ache des Objektivs steht insbesondere senkrecht auf einer Abschlussebene des Schafts an dem distalen Ende. Diese Abschlussebene kann wie bei aus dem Stand der Technik bekannten Endoskopen gegenüber einer Ebene senkrecht zu der Längsachse geneigt sein. Die Abschlussebene wird optional durch den ersten Anlageabschnitt, den zweiten Anlageabschnitt, das Verbindungselement, das Bündel von Lichtleitern, das Objektiv und/oder das Mantelrohr gebildet. Insbesondere sind diese Elemente komplett bündig zu der Abschlussebene angeordnet.

Der erste Anlageabschnitt weist in Draufsicht auf das distale Ende eine Innenfläche, die optional als Objektivaufnahme ausgestaltet ist, und eine Außenfläche auf. Ein Teil der Außenfläche bildet die erste Anlagefläche, wobei ein Bereich der ersten Anlagefläche zu der zweiten Anlagefläche beabstandet und gegenüberliegend angeordnet ist. Um die gegenseitige Anordnung der beiden Anlageflächen dauerhaft festzulegen, so dass eine bessere Anordnung des Bündels von Lichtleitern möglich ist, sind der erste Anlageabschnitt und der zweite Anlageabschnitt durch das Verbindungselement miteinander verbunden. Das Verbindungselement kann jede beliebige Form annehmen, so lange es eine dauerhafte und stabile Verbindung zwischen dem ersten Anlageabschnitt und dem zweiten Anlageabschnitt ermöglicht. Das Verbindungselement kann bündig zu der Abschlussebene angeordnet sein; es ist jedoch auch möglich, dass das Verbindungselement von der Abschlussebene beabstandet ist, beispielsweise ist das Verbindungselement im Inneren des Schafts angeordnet.

Auf diese Weise wird das Bündel von Lichtleitern über eine große Fläche durch die erste Anlagefläche und die zweite Anlagefläche begrenzt, so dass das Bündel von Lichtleitern besonders einfach in das Mantelrohr eingeführt werden kann. Beispielsweise wird in Draufsicht auf das distale Ende das Bündel von Lichtleitern lediglich durch die erste Anlagefläche, das Verbindungselement, die zweite Anlagefläche und das Mantelrohr begrenzt. Vorzugsweise ist dabei der Anteil des Mantelrohrs im Vergleich zu den übrigen Elementen gering; es ist bevorzugt, dass das Bündel von Lichtleitern an dem distalen Ende lediglich von dem Verbindungselement, der ersten Anlagefläche und der zweiten Anlagefläche begrenzt wird. Je nach Anzahl der Lichtleiter liegen alle oder eine Teilmenge der Lichtleiter an der ersten und/oder der zweiten Anlagefläche an. Beispielsweise, wenn viele dünne Lichtleiter vorgesehen sind, liegen nur die jeweils äußeren Lichtleiter des Bündels an den Anlageflächen an.

Bereiche der ersten Anlagefläche und/oder der zweiten Anlagefläche sind nicht-parallel zu der Längsachse angeordnet, d.h. diese Bereiche der ersten und/oder zweiten Anlagefläche schließen einen Winkel mit der Längsachse ein. Dieser Winkel wird insbesondere in einer Ebene senkrecht zu den entsprechenden Bereichen der ersten und/oder zweiten Anlagefläche bestimmt. Dadurch, dass das Bündel von Lichtleitern an der ersten Anlagefläche und der zweiten Anlagefläche anliegt, wird wenigstens ein Teil der Lichtleiter aus deren Verlauf entlang der Längsachse ausgelenkt, so dass die Richtung der hauptsächlichen Beleuchtung des ausgelenkten Teils der Lichtleiter nicht-parallel zu der Längsachse ist.

Um ein Verschließen des distalen Endes des Schafts zu erleichtern und das Anordnen des Bündels von Lichtleitern an dem distalen Ende zu vereinfachen, ist es bevorzugt, dass der erste Anlageabschnitt und/oder der zweite Anlageabschnitt an einer Innenfläche des Mantelrohrs anliegen. Der erste Anlageabschnitt und/oder der zweite Anlageabschnitt liegen in Umfangsrichtung des Mantelrohrs vorzugsweise bündig an diesem an. Insbesondere liegt der Teil der Außenfläche des ersten Anlageabschnitts, welcher nicht als erste Anlagefläche dient, an der Innenfläche des Mantelrohrs an. Der erste Anlageabschnitt kann ringförmig ausgebildet sein. Der zweite Anlageabschnitt kann sichelförmig ausgebildet sein. Der zweite Anlageabschnitt weist in Draufsicht auf das distale Ende optional lediglich die zweite Anlagefläche und eine Fläche auf, mittels welcher dieser an der Innenfläche des Mantelrohrs anliegt.

Darüber hinaus erlaubt die Weiterbildung des Endoskops, wonach der erste Anlageabschnitt und der zweite Anlageabschnitt an einer Innenfläche des Mantelrohrs anliegen, eine besonders präzise Anordnung des Bündels von Lichtleitern, da durch das Anliegen des ersten und zweiten Anlageabschnitts das Bündel von Lichtleitern hinsichtlich der Ausrichtung gegenüber der Längsachse vorgegeben ist. Dazu ist es bevorzugt, dass die Flächen, an denen der erste Anlageabschnitt und der zweite Anlageabschnitt an dem Innenrohr des Mantelrohrs anliegen, sich gegenüberliegen, so dass lediglich die Orientierung entlang der Umfangsrichtung festgelegt werden muss.

Um die durch das Endoskop bereitgestellte Beleuchtung zu optimieren, ist es bevorzugt, dass das Endoskop ein Objektiv mit einer optischen Achse aufweist, welche mit der Längsachse einen Winkel einschließt, der gleich einem Winkel ist, welcher die Längsachse mit dem Bereich der ersten Anlagefläche und/oder dem Bereich der zweiten Anlagefläche einschließt. Dies bedeutet, dass die optische Achse in einer Ebene parallel zu dem geneigten Bereich der ersten Anlagefläche und/oder der zweiten Anlagefläche verläuft. Dadurch wird erreicht, dass die einzelnen Lichtleiter, die an dem geneigten Bereich der ersten und/oder zweiten Anlagefläche anliegen, parallel zur optischen Achse verlaufen. Dadurch ist die Richtung der Beleuchtung parallel zur optischen Achse des Objektivs, so dass der mit dem Objektiv beobachtete Bereich besonders gut ausgeleuchtet ist.

Um eine homogene Beleuchtung des mit dem Endoskop zu beobachtenden Objekts bereitzustellen, ist es bevorzugt, dass Teilbereiche der ersten Anlagefläche und/oder der zweiten Anlagefläche unterschiedlich stark gegen die Längsachse geneigt sind, wobei vorzugsweise der Winkel in Umfangsrichtung zunimmt. Beispielsweise weisen die erste Anlagefläche und/oder die zweite Anlagefläche zwei oder mehr Teilbereiche auf, die unterschiedlich stark gegenüber der Längsachse geneigt sind. Optional weist lediglich die erste Anlagefläche Teilbereiche auf, die unterschiedlich stark geneigt sind. Beispielsweise ist ein Teilbereich der ersten Anlagefläche, welcher der zweiten Anlagefläche nicht gegenüber liegend angeordnet ist, stärker gegenüber der Längsachse geneigt, als der Teilbereich, welcher der zweiten Anlagefläche gegenüberliegt. Die erste Anlagefläche und/oder zweite Anlagefläche kann die Neigung gegenüber der Längsachse graduell ändern, insbesondere entlang der Umfangsrichtung des Schafts.

In einer Weiterbildung des Endoskops sind zwei Teilbereiche der ersten Anlagefläche vorgesehen, die stärker gegenüber dem Bereich der ersten Anlagefläche, der der zweiten Anlagefläche gegenüberliegt, geneigt und diese sind optional auf gegenüberliegenden Seiten des Objektivs angeordnet. Diese gegenüberliegenden Seiten sind vorzugsweise auf einer Höhe der optischen Achse angeordnet, wobei der Bereich der ersten Anlagefläche, der der zweiten Anlagefläche gegenüberliegt, höher, also in einer Höhenrichtung weiter entfernt von der optischen Achse, angeordnet ist. Aufgrund der größeren Neigung kann erreicht werden, dass die Lichtleiter nicht nur eine Beleuchtung um das Objektiv bereitstellen, sondern auch andere Bereiche ausleuchten. Damit lässt sich eine Verteilung der Richtung der Beleuchtung für die einzelnen Lichtleiter erzielen, wodurch das Objekt, das mit dem Endoskop betrachtet werden soll, homogener ausgeleuchtet werden kann.

Die Bereiche der ersten Anlagefläche und der zweiten Anlagefläche, welche sich gegenüberliegen, können so gegen die Längsachse geneigt sein, dass ihr Abstand in Richtung zum distalen Ende hin abnimmt.

Ferner kann ein durch den ersten und zweiten Anlageabschnitt begrenzter Raum zum distalen Ende hin kleiner werden. Man kann auch sagen, dass der begrenzte Raum zum proximalen Ende des Endoskops aufgeht.

Um die Anordnung der Lichtbündel weiter zu vereinfachen, werden die Lichtleiter an dem distalen Ende durch das Verbindungselement in mindestens zwei Teilbündel getrennt. Auf diese Weise kann eine Teilmenge der Lichtleiter separat ausgerichtet werden, wodurch sich die Anzahl der jeweils anzuordnenden Lichtleiter reduziert. Dies vereinfacht die Anordnung, wobei es gleichzeitig weiterhin möglich ist, die gesamte Anzahl von Lichtleitern anzuordnen, jedoch jeweils in Zwischenschritten. Diese Trennung gelingt besonders gut dann, wenn das Verbindungselement, insbesondere der Steg, mittig angeordnet ist, so dass sich zwei Kammern ausbilden, wobei jede Kammer durch das Verbindungselement, die erste Anlagefläche, die zweite Anlagefläche sowie optional durch die Innenseite des Mantelrohrs begrenzt wird.

Eine besonders homogene Beleuchtung des Objekts lässt sich erreichen, indem der Innenrohrkopf in Draufsicht auf das distale Ende symmetrisch, optional zu einer durch das Verbindungselement verlaufenden Symmetrieachse oder einer Symmetrieebene, in der das Verbindungselement und die Längsachse liegen, ist. Insbesondere bildet der Steg die Symmetrieachse. Dies bedeutet, dass auf jeder Seite der Symmetrieachse die Lichtleiter identisch angeordnet sind, so dass das Objekt symmetrisch, vorzugsweise zu der Symmetrieachse, ausgeleuchtet wird. Insbesondere sind dazu die erste Anlagefläche und die zweite Anlagefläche auf jeder Seite der Symmetrieachse identisch ausgebildet. Vorzugsweise verläuft die optische Achse des Objektivs in der Symmetrieebene. Auf diese Weise ist die Beleuchtung besonders gut auf das Objektiv ausgerichtet.

Darüber hinaus wird ein Verfahren zum Fixieren eines Bündels von Lichtleitern in einem Schaft eines Endoskops bereitgestellt, welches die Schritte des Anspruches 10 umfasst. Optional kann das distale Ende beschliffen werden.

Der Innenrohrkopf kann einen Vorsprung, der in Richtung der Längsachse sowie senkrecht zu der Längsachse vorsteht, aufweisen und das Einführen des Bündels von Lichtleitern mit dem Innenrohr in das Mantelrohr kann so durchgeführt werden, dass der Vorsprung in Richtung der Längsachse sowie senkrecht zu der Längsachse vorsteht und dass der Vorsprung in einer Ausnehmung an dem distalen Ende des Mantelrohrs angeordnet wird. Die Ausnehmung kann sich entlang der Längsachse erstrecken und/oder bis zum distalen Ende erstrecken, so dass sie zum distalen Ende hin offen ist.

Vorteil des Vorsehens des Vorsprungs und der Ausnehmung ist, dass der Innenrohrkopf in Umfangsrichtung besonders einfach positioniert werden kann. Die Ausrichtung in Umfangsrichtung wird durch den Vorsprung und die Ausnehmung bestimmt. Liegen der erste Anlageabschnitt und der zweite Anlageabschnitt ferner an der Innenfläche des Mantelrohrs an, so wie dies zuvor beschrieben wurde, ist die Positionierung des Innenrohrkopfs in dem Mantelrohr eindeutig festgelegt, so dass Fehler beim Herstellen des Endoskops vermieden werden können.

Der Vorsprung kann an dem ersten Anlageabschnitt oder an dem zweiten Anlageabschnitt angeordnet sein. Der Vorsprung ist vorzugsweise einstückig mit dem Innenrohrkopf ausgebildet. Der Vorsprung steht insbesondere nur in dem Bereich, in dem er in Richtung der Längsachse vorsteht, senkrecht zu der Längsachse vor; optional steht der Vorsprung von dem Innenrohrkopf in Richtung der optischen Achse des Objektivs vor. Wird die Abschlussebene durch den ersten Anlageabschnitt und/oder den zweiten Anlageabschnitt gebildet, ragt der Vorsprung aus der Abschlussebene hervor. Dadurch, dass der Vorsprung senkrecht zur Längsachse, d.h. in radialer Richtung des Mantelrohrs vorsteht, kann dieser in die Ausnehmung des Mantelrohrs eingeführt werden, um den Innenrohrkopf in der Umfangsrichtung zu fixieren. Die Ausnehmung ist an dem distalen Ende des Mantelrohrkopfs vorgesehen. Der Vorsprung passt vorzugsweise bündig in die Ausnehmung.

Das Fixieren des Bündels von Lichtleitern erfolgt insbesondere durch das Aufbringen von Klebstoff, so wie dies zuvor beschrieben wurde. Insbesondere gelten die in dem Zusammenhang mit dem Endoskop angestellten Überlegungen, bevorzugte Ausführungsformen und Vorteile analog für das Verfahren.

Um das distale Ende des Schafts zuverlässig abzudichten, ist es bevorzugt, dass das distale Ende derart beschliffen wird, dass der Vorsprung in seiner Ausdehnung in Richtung der Längsachse entfernt ist. Dadurch dass der Vorsprung vorzugsweise nur im Bereich, in welchem er in Richtung der Längsachse vorsteht, auch senkrecht zu der Längsachse vorsteht, wird durch das Beschleifen des Vorsprungs in Richtung der Längsachse der Vorsprung komplett abgeschliffen. Gleichzeitig wird auch die Ausnehmung weggeschliffen, da der Vorsprung in die Ausnehmung hineinragt. Nach dem Schleifen sind somit der Vorsprung und die Ausnehmung verschwunden, so dass sich die Fläche, die abgedichtet werden muss, verringert. Insbesondere wird das distale Ende parallel zu der gewünschten Abschlussebene beschliffen.

Die zuvor angestellten Überlegungen, bevorzugte Weiterbildungen und Vorteile gelten analog für dieses Verfahren.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine Seitenansicht eines Endoskops;
- Fig. 2: eine Draufsicht auf ein distales Ende des Endoskops gemäß Fig. 1;
- Fig. 3: eine Querschnittansicht des Endoskops entlang der Linie A-A von Fig. 2;
- Fig. 4A: eine perspektivische Ansicht des Endoskops von Fig. 1 von der Seite;
- Fig. 4B: eine vergrößerte Darstellung des Details B von Fig. 4A;
- Fig. 5A: eine perspektivische Ansicht des Endoskops von Fig. 1 von oben;
- Fig. 5B: eine vergrößerte Darstellung des Details C von Fig. 5A;
- Fig. 6A: eine Seitenansicht eines Innenrohrs des Endoskops von Fig. 1;
- Fig. 6B: eine vergrößerte Darstellung des Details D von Fig. 6A;
- Fig. 7A: eine perspektivische Ansicht des Innenrohrs von Fig. 6 von schräg unten;
- Fig. 7B: eine vergrößerte Darstellung des Details E von Fig. 7A;

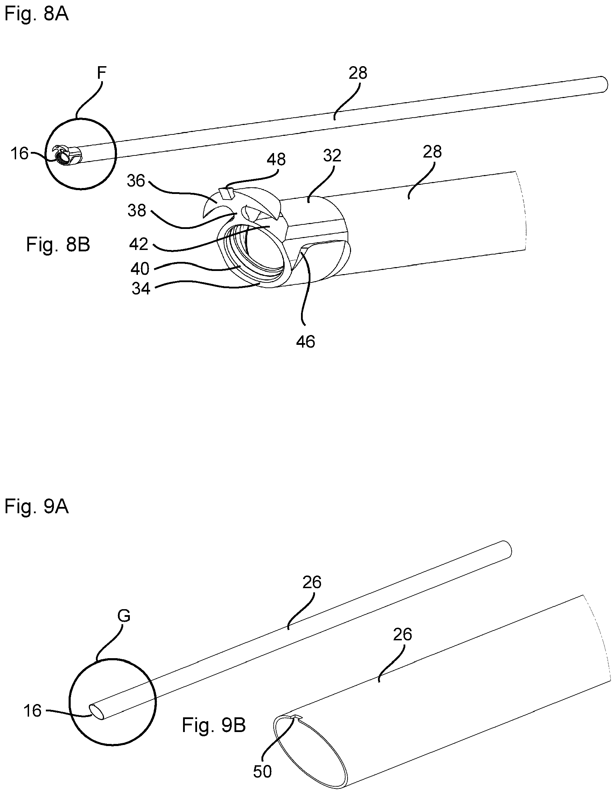

- Fig. 8A: das Innenrohr gemäß den Fig. 6 und 7, bevor es in dem Endoskop verbaut wurde;
- Fig. 8B: eine vergrößerte Darstellung des Details F von Fig. 8A;
- Fig. 9A: eine perspektivische Darstellung eines Mantelrohrs des in Fig. 1 dargestellten Endoskops, bevor es in dem Endoskop verbaut wurde; und
- Fig. 9B: eine vergrößerte Darstellung des Details G von Fig. 9A.

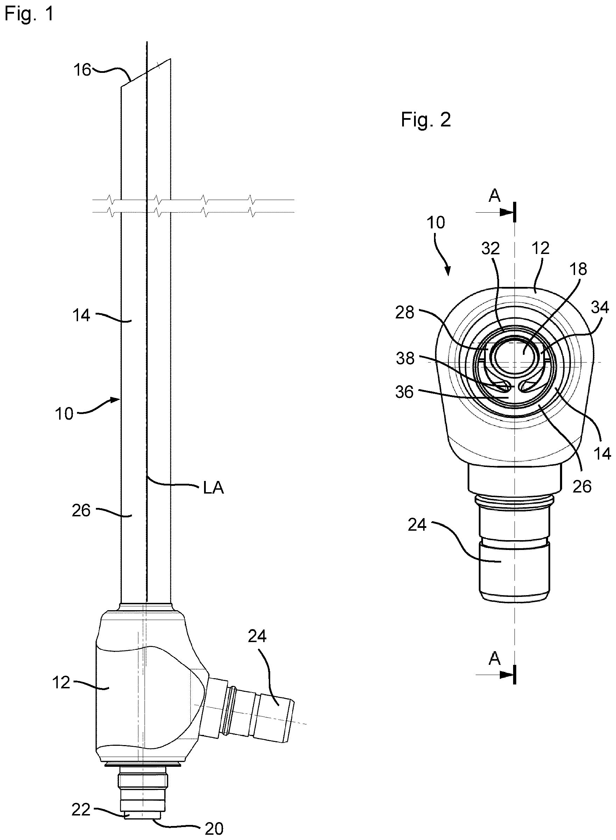

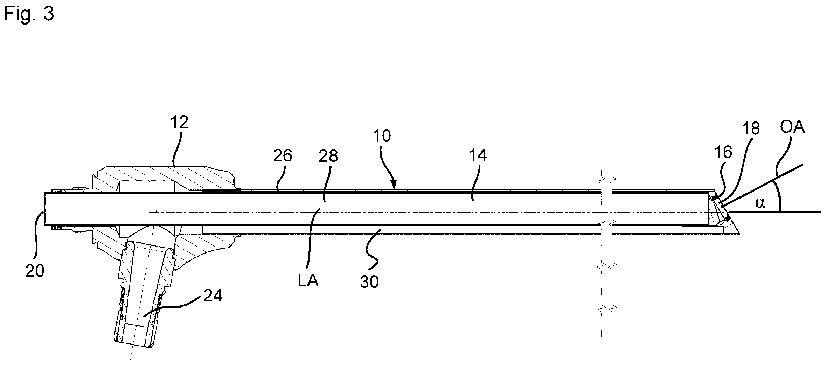

Ein Endoskop 10 weist ein Hauptteil 12 und einen Schaft 14 auf. Das Hauptteil 12 ist mit dem Schaft 14 verbunden, beispielsweise wird die Verbindung mittels Fügen, wie Hartlöten oder Schweißen realisiert. Der Schaft 14 erstreckt sich entlang einer Längsachse LA. Das Endoskop 10 weist ein distales Ende 16 auf, an welchem ein Objektiv 18 vorgesehen ist, wie dies insbesondere in den Fig. 2 und 3 sichtbar ist. An einem proximalen Ende 20 des Endoskops 10 ist ein Okular 22 vorgesehen. Mithilfe des Objektivs 18 und des Okulars 22 und eventuell weiterer, nicht dargestellter optischer Elemente, lässt sich ein Objekt, das vor dem distalen Ende angeordnet ist, durch das Endoskop 10 abbilden. So kann das distale Ende 16 des Endoskops 10 beispielsweise in eine Körperöffnung eingeführt, so dass das Innere der Körperöffnung mittels des Okulars 22 betrachtet werden kann.

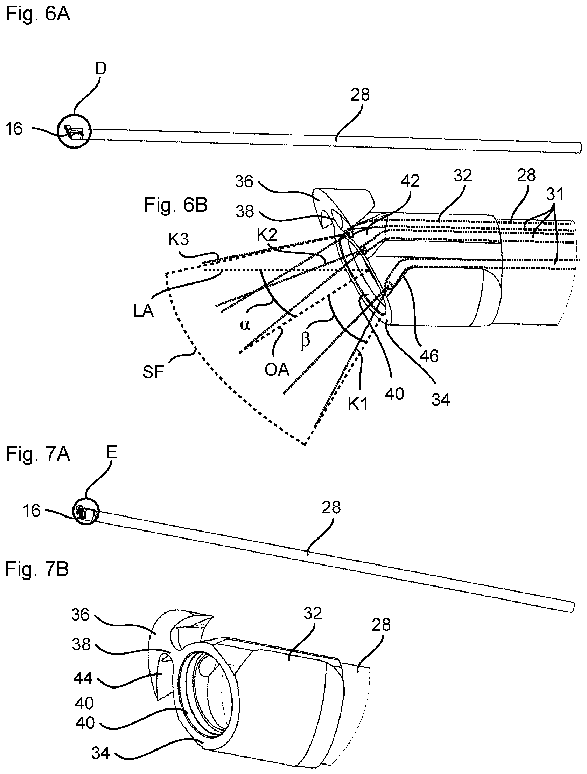

An dem Hauptteil 12 ist ein optischer Anschluss 24 für eine (nicht gezeigte) Lichtquelle vorgesehen. Von dem optischen Anschluss 24 erstreckt sich ein Bündel von Lichtleitern 31 durch das Hauptteil 12 und den Schaft 14 bis zum distalen Ende 16. Zur Vereinfachung der Darstellungen sind die Lichtleitern 31 lediglich in Fig. 6B schematisch eingezeichnet. Obwohl in Fig. 6B drei Lichtleitern 31 dargestellt sind, weist das Bündel von Lichtleitern 31 in der Regel sehr viel mehr Lichtleitern 31 auf (z. B. einige hundert oder einige tausend). Die Lichtleiter 31 dienen dazu, das Licht der mit dem optischen Anschluss 24 verbundenen Lichtquelle bis zum distalen Ende 16 zu führen und dort zur Beleuchtung des zu beobachtenden Objektes abzugeben.

Wie dies insbesondere in Fig. 3 zu erkennen ist, weist der Schaft 14 ein Mantelrohr 26 und ein Innenrohr 28 auf, welche beide entlang der Längsachse LA verlaufen. Das Innenrohr 28 ist in dem Mantelrohr 26 angeordnet. Das Innenrohr 28 weist einen Außenrohrdurchmesser auf, welcher kleiner ist als ein Innenrohrdurchmesser des Mantelrohrs 26. Dadurch ergibt sich ein Hohlraum 30, in welchem das Bündel von Lichtleitern 31 angeordnet werden kann. Das Bündel von Lichtleitern 31 erstreckt sich von dem optischen Anschluss 24 durch den Schaft 14 entlang der Längsachse LA bis zu dem distalen Ende 16. Dort sind die Lichtleiter 31 von der Längsachse LA ausgelenkt, so dass sie das Objekt beleuchten (siehe Fig. 6B). In dem Innenrohr 28 können nicht gezeigte Stablinsen angeordnet sein, welche eine optische Verbindung zwischen dem Objektiv 18 und dem Okular 22 bereitstellen.

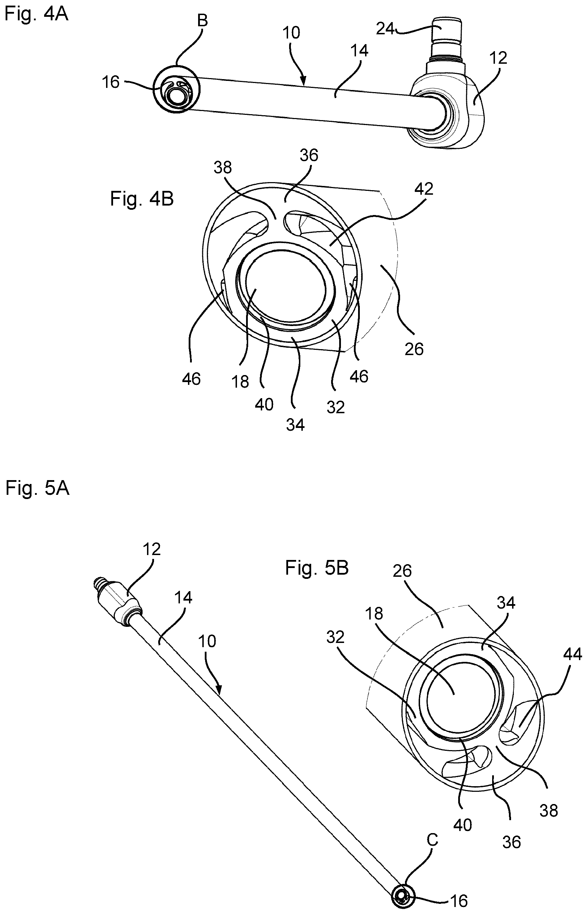

Das Innenrohr 28 weist an dem distalen Ende 16, d.h. einem dem Hauptteil 12 abgewandten Ende, einen Innenrohrkopf 32 auf. Das Objektiv 18 wird durch das Innenrohr 28, insbesondere durch den Innenrohrkopf 32, fixiert. Wie dies insbesondere in den Fig. 4-7 zu erkennen ist, weist der Innenrohrkopf 32 einen ersten Anlageabschnitt 34, einen zweiten Anlageabschnitt 36 und ein Verbindungselement 38 auf. Der Innenrohrkopf 32 kann einstückig mit dem Innenrohr 28 ausgebildet sein. Bevorzugt ist jedoch, dass der Innenrohrkopf 32 mithilfe eines Fügeverfahrens dauerhaft mit dem Innenrohr 28 verbunden wird, beispielsweise mittels Hartlöten, Kleben oder Schweißen. Das Mantelrohr 26, das Innenrohr 28 und/oder der Innenrohrkopf 32 sind aus einem metallischen Werkstoff, beispielsweise Edelstahl, hergestellt.

Der erste Anlageabschnitt 34 weist eine Objektivaufnahme 40 und eine erste Anlagefläche 42 auf. Die Objektivaufnahme 40 kann in Draufsicht wie eine kreisrunde Öffnung ausgebildet sein, in welcher das Objektiv 18 fixiert werden kann. Die Objektivaufnahme 40 ist derart angeordnet, dass eine optische Achse OA des Objektivs 18 gegenüber der Längsachse LA geneigt ist und einen Winkel α einschließt (Fig. 3; Fig. 6B). Auf diese Weise ist es möglich, dass mit dem Endoskop 10 das Objekt seitlich zur Längsachse LA betrachtet werden kann, d.h. dass nicht nur entlang der Längsachse LA Objekte mittels des Endoskops 10 betrachtet werden können. Die optische Achse OA legt somit einen Blickwinkel fest. Ein Öffnungswinkel β des Objektivs 18, der zusammen mit dem Blickwinkel ein Sichtfeld SF des Endoskops 10 festlegt, kann beispielsweise ±5°, ±10°, ±15°, ±20°, ±25°, ±30°, ±35° oder ±37,5° um die optische Achse OA betragen. Das Sichtfeld SF ist beispielhaft in Fig. 6B dargestellt. Die erste Anlagefläche 42 befindet sich auf einer Außenfläche des ersten Anlageabschnitts 34. Ein weiterer Abschnitt der Außenfläche des ersten Anlageabschnitts 34 liegt an dem Mantelrohr 26, vorzugsweise entlang dessen Umfangsrichtung bündig, an. An der ersten Anlagefläche 42 liegt das Bündel von Lichtleitern 31 an, welche, wie erwähnt, in den Figuren nicht dargestellt sind.

Der zweite Anlageabschnitt 36 liegt ebenfalls, vorzugsweise in Umfangsrichtung des Mantelrohrs 26 bündig, an diesem an. Darüber hinaus weist der zweite Anlageabschnitt 36 eine zweite Anlagefläche 44 auf, an welcher ebenfalls das Bündel von Lichtleitern 31 anliegt. Die erste Anlagefläche 42 und die zweite Anlagefläche 44 sind zumindest bereichsweise gegenüber der Längsachse LA nicht-parallel angeordnet. Dies bedeutet, dass Bereiche der ersten Anlagefläche 42 und/oder der zweiten Anlagefläche 44 einen Winkel mit der Längsachse LA einschließen. Diese Bereiche der ersten Anlagefläche 42 und der zweiten Anlagefläche 44 liegen sich gegenüber. Vorzugsweise verlaufen die erste Anlagefläche 42 und/oder die zweite Anlagefläche 44 bereichsweise parallel zu der optischen Achse OA des Objektivs 18, so dass der erwähnte Winkel gleich dem Winkel α zwischen der optischen Achse OA und der Längsachse LA ist.

Die erste Anlagefläche 42 weist darüber hinaus einen Teilbereich 46 auf, der stärker gegenüber der Längsachse LA als der übrige Abschnitt der ersten Anlagefläche 42 geneigt ist. Dies bedeutet, dass der Winkel, welcher die Fläche des Teilbereichs 46 mit der Längsachse LA einschließt, größer ist als der Bereich der ersten Anlagefläche 42 welcher der zweiten Anlagefläche 44 gegenüberliegt. Der Teilbereich 46 ist auf beiden Seiten des ersten Anlageabschnitts 34 bezogen auf das Objektiv 18 vorgesehen und liegt der zweiten Anlagefläche 44 nicht gegenüber. Die erste Anlagefläche 42 und/oder zweite Anlagefläche 44 kann die Neigung gegenüber der Längsachse LA graduell ändern, insbesondere entlang der Umfangsrichtung des Schafts 14. Der Neigungswinkel der entsprechenden Anlagefläche 42, 44 oder von Teilabschnitten der Anlagefläche 42, 44 kann somit entlang der Umfangsrichtung zunehmen. Insbesondere kann die Zunahme in Richtung weg vom Steg 38 stattfinden.

Wie dies insbesondere in Fig. 6B dargestellt ist, wird dadurch eine unterschiedliche Ablenkung der Lichtleiter 31 aus der Längsachse LA erreicht. Wie dies beispielhaft in Fig. 6B anhand dreier Lichtleiter 31 dargestellt ist, weisen die von den Lichtleitern 31 erzeugten Lichtkegel K1, K2 und K3 und somit die entsprechenden Hauptstrahlrichtungen jeweils eine andere Richtung auf, d.h. der Winkel der Hauptstrahlrichtung gegenüber der Längsachse LA ist für jeden der drei Lichtleiter 31 anders. Dies bedeutet, dass nicht jeder Lichtleiter 31 gleich relativ zu der optischen Achse OA positioniert ist. Die durch die Lichtleiter 31 beleuchtete Fläche des Objekts bildet somit keinen Ring oder Kranz um die optische Achse OA. Vielmehr wird das Sichtfeld SF und somit das Objekt homogen ausgeleuchtet. Bei der schematischen Darstellung in Fig. 6B überlappen beispielsweise die Lichtkegel K2 und K3. Insbesondere ist auch bei einem großen Sichtfeld SF des Endoskops 10 eine sehr homogene Ausleuchtung möglich.

Das Verbindungselement 38 ist in der gezeigten Ausführungsform als Steg ausgebildet, von welchem die erste Anlagefläche 42 und die zweite Anlagefläche 44 vorstehen. Das Verbindungselement 38 dient dazu, den zweiten Anlageabschnitt 36 dauerhaft mit dem ersten Anlageabschnitt 34 zu verbinden. Der Innenrohrkopf 32 kann somit als einstückige Einheit ausgebildet sein.

Der Innenrohrkopf 32 kann symmetrisch ausgebildet sein. Beispielsweise schneidet die Symmetrieachse in Draufsicht auf das distale Ende 16 die optische Achse OA und die Längsachse LA. Ist das Verbindungselement 38 als Steg ausgebildet, kann die Symmetrieachse durch den Steg 38 verlaufen. Die erste Anlagefläche 42, die zweite Anlagefläche 44 und das Verbindungselement 38 bilden in Draufsicht auf das distale Ende 16 eine Doppel-T-Form.

Der erste Anlageabschnitt 34 und der zweite Anlageabschnitt 36 sowie optional das Verbindungselement 38 und/oder das Mantelrohr 26 begrenzen eine oder mehrere Kammern (z. B. einseitig offene Kammern), in welchen das Bündel von Lichtleitern 31 angeordnet werden kann. In der gezeigten Ausführungsform, bei der das Verbindungselement 38 ein auf der Symmetrieachse angeordneter Steg ist, werden zwei Kammern zur Anordnung des Bündels von Lichtleitern 31 bereitgestellt. Jede Kammer nimmt daher ungefähr die Hälfte der Lichtleiter 31 auf. Jede Kammer wird in der gezeigten Ausführungsform durch das Mantelrohr 26, den ersten Anlageabschnitt 34, das Verbindungselement 38 und den zweiten Anlageabschnitt 36 begrenzt.

Die Herstellung des Endoskops 10 ist wie folgt:
Der Innenrohrkopf 32 und das Mantelrohr 26, wie sie in Fig. 1-7 dargestellt sind, zeigen deren Ausgestaltung in dem fertigen Endoskop 10. Vor der Fertigstellung weist der Innenrohrkopf 32 einen Vorsprung 48 auf und das Mantelrohr 26 eine Ausnehmung 50. Der Vorsprung 48 kann an dem zweiten Anlageabschnitt 36 vorgesehen sein; es ist jedoch auch möglich, dass der Vorsprung 48 an den ersten Anlageabschnitt 34 angeordnet wird. Der Vorsprung 48 steht in Richtung der Längsachse LA und senkrecht zu der Längsachse LA von dem Innenrohrkopf 32, insbesondere von dem zweiten Anlageabschnitt 36, vor. Der Vorsprung 48 steht nur im Bereich, in welchem er von dem Innenrohrkopf 32 in Richtung der Längsachse LA, insbesondere der optischen Achse OA, vorsteht senkrecht zu der Längsachse LA hervor. Der Vorsprung 48 ist demnach so ausgestaltet, als ob er auf dem zweiten Anlageabschnitt 36 aufgebracht wurde.

Zur Herstellung des Endoskops 10 wird das Innenrohr 28 mit dem Innenrohrkopf 32 bereitgestellt, beispielsweise dadurch, dass der Innenrohrkopf 32 mit dem Innenrohr 28 verbunden wird. Dann wird an dem Innenrohrkopf 32 das Objektiv 18 montiert. Im Anschluss daran wird das Bündel von Lichtleitern 31 an dem ersten Anlageabschnitt 34 und dem zweiten Anlageabschnitt 36 vorgesehen. Insbesondere wird das Bündel von Lichtleitern 31 zwischen der ersten Anlagefläche 42 und der zweiten Anlagefläche 44 eingeklemmt. Dadurch, dass die erste Anlagefläche 42 und die zweite Anlagefläche 44 gegenüber der Längsachse LA geneigt sind, wird ein distales Ende 16 des Bündels von Lichtleitern 31 gegenüber der Längsachse LA ausgebogen.

Im Anschluss daran wird das Innenrohr 28 mit dem Innenkopf 32 sowie dem Bündel von Lichtleitern 31 in das Mantelrohr 26 eingeführt. Dadurch, dass das Bündel von Lichtleitern 31 zwischen dem ersten Anlageabschnitt 34 und dem zweiten Anlageabschnitt 36 verklemmt ist, gelingt dies auf besonders einfache Weise. Abgesehen von dem distalen Ende 16 verläuft das Bündel von Lichtleitern 31 parallel zu der Längsachse LA; an dem distalen Ende 16 ist das Bündel von Lichtleitern 31 aufgrund der Neigung der ersten Anlagefläche 42 und der zweiten Anlagefläche 44 gegenüber der Längsachse LA geneigt. Die Neigung gegenüber der Längsachse L ist unterschiedlich, da der Teilbereich 46 eine andere Neigung gegenüber der Längsachse LA hat als der Rest der ersten Anlagefläche 42.

Beim Einführen des Innenrohrs 28 wird darauf geachtet, dass der Vorsprung 48 in die Ausnehmung 50 eingeführt wird. Auf diese Weise gelingt es, den Innenrohrkopf 32 und damit dessen Auslenkung gegenüber der Längsachse LA so anzuordnen, dass die Neigung des Innenrohrkopfs 32 mit der Neigung des distalen Endes 16 des Mantelrohrs 26 übereinstimmt.

Im Anschluss daran wird das Bündel von Lichtleitern 31 in dem Innenrohrkopf 32 und dem Mantelrohr 26 fixiert. Dies wird beispielsweise dadurch erreicht, dass die genannten Teile mittels Klebstoff miteinander verbunden werden. Auf diese Weise wird auch das distale Ende 16 abgedichtet. Anschließend wird das distale Ende 16 beschliffen und zwar in einer Ebene senkrecht zu der optischen Achse OA des Objektivs 18. Das distale Ende 16 wird so lange beschliffen, bis der Vorsprung 48 entlang der Längsachse LA, insbesondere der optischen Achse OA des Objektivs 18, nicht mehr von den Innenrohrkopf 32 hervorsteht. Der Vorsprung 48 ist somit bündig zu dem Innenrohrkopf 32, insbesondere zu dem zweiten Anlageabschnitt 36, abgeschliffen worden.

Durch dieses Abschleifen ist der Vorsprung 48 komplett verschwunden, d.h. er steht auch nicht mehr senkrecht zu der Längsachse LA hervor, da er nur in dem Bereich, in welchem er in Richtung der Längsachse LA, insbesondere der optischen Achse OA, vorsteht auch senkrecht zu der Längsachse LA hervorsteht. Gleichzeitig wurde auch das Mantelrohr 26 derart am distalen Ende 16 beschliffen, dass die Ausnehmung 50 verschwunden ist. Es ergibt sich somit der in den Fig. 1-7 gezeigte Aufbau des distalen Endes 16 des Endoskops 10.

Durch die Neigung der ersten Anlagefläche 42 und/oder der zweiten Anlagefläche 44 gegenüber der Längsachse LA kann das mit dem Endoskop 10 zu beobachtende Objekt beleuchtet werden. Die Beleuchtung ist dabei insbesondere parallel zu der optischen Achse OA, so dass die Hauptintensität der von dem Bündel von Lichtleitern 31 emittierten Strahlung tatsächlich auf das von dem Endoskop 10 zu beobachtende Objekt fällt. Die Bereitstellung des Teilbereichs 46, der eine größere Neigung gegenüber der Längsachse LA als der Rest der ersten Anlagefläche 42 hat, hilft dabei, auch die Seiten des Objekts zu beleuchten, die von der zweiten Anlagefläche 44 abgewandt sind, d.h. in denen keine Lichtleiter 31 angeordnet sind. Auf diese Weise kann das Sichtfeld des Endoskops 10, welches durch den Winkel α der optischen Achse OA und den Öffnungswinkel um die optische Achse OA festgelegt ist, gleichmäßig beleuchtet werden, da die einzelnen Lichtleiter 31 an dem distalen Ende 16 in unterschiedliche Richtungen weisen.

## Patentansprüche

1. Endoskop mit einem Hauptteil (12) und einem mit dem Hauptteil (12) verbundenen Schaft (14), welcher sich entlang einer Längsachse (LA) erstreckt,
- wobei der Schaft (14) ein Mantelrohr (26), ein in dem Mantelrohr (26) angeordnetes Innenrohr (28) zur Aufnahme eines Objektivs (18) und ein Bündel von Lichtleitern (31), die in dem Schaft (14) zwischen dem Mantelrohr (26) und dem Innenrohr (28) entlang der Längsachse (LA) verlaufen, umfasst,
- wobei das Innenrohr (28) an einem von dem Hauptteil (12) abgewandten, distalen Ende (16) einen Innenrohrkopf (32) aufweist, welcher einen ersten Anlageabschnitt (34) und einen zweiten Anlageabschnitt (36), der mit dem ersten Anlageabschnitt (34) durch ein Verbindungselement (38) verbunden ist, umfasst,
- wobei der erste Anlageabschnitt (34) eine erste Anlagefläche (42), an dem das Bündel von Lichtleitern (31) anliegt, aufweist und der zweite Anlageabschnitt (36) eine zweite Anlagefläche (44), an dem das Bündel von Lichtleitern (31) anliegt und welche der ersten Anlagefläche (42) wenigstens bereichsweise gegenüberliegt, aufweist,
- wobei zur Ausrichtung des Bündels von Lichtleitern (31) aus der Längsachse (LA) an dem distalen Ende (16) ein Bereich der ersten Anlagefläche (42) und/oder ein Bereich der zweiten Anlagefläche (44) nicht-parallel zu der Längsachse (LA) verlaufen, wobei das Verbindungselement (38) als Steg ausgebildet ist,
wobei das Bündel von Lichtleitern (31) an dem distalen Ende (16) durch das Verbindungselement (38) in mindestens zwei Teilbündel getrennt ist und der erste Anlageabschnitt (34), das Verbindungselement (38) und der zweite Anlageabschnitt (36) in Draufsicht auf das distale Ende (16) eine Doppel-T-Form bilden.

2. Endoskop nach Anspruch 1, **dadurch gekennzeichnet, dass** der erste Anlageabschnitt (34) und/oder der zweite Anlageabschnitt (36) an einer Innenfläche des Mantelrohrs (26) anliegen.

3. Endoskop nach Anspruch 1 oder 2, **gekennzeichnet durch** ein Objektiv (18), das eine optische Achse (OA) aufweist, welche mit der Längsachse (LA) einen Winkel (α) einschließt, der gleich einem Winkel ist, welcher die Längsachse (LA) mit dem Bereich der ersten Anlagefläche (42) und/oder dem Bereich der zweiten Anlagefläche (44) einschließt.

4. Endoskop nach einem der obigen Ansprüche, **dadurch gekennzeichnet, dass** Teilbereiche (46) der ersten Anlagefläche (42) und/oder der zweiten Anlagefläche (44) unterschiedlich stark gegen die Längsachse (LA) geneigt sind, wobei vorzugsweise der Neigungswinkel der Teilbereiche (46) entlang einer Umfangsrichtung des Schaftes (4) zunimmt.

5. Endoskop nach einem der obigen Ansprüche, **dadurch gekennzeichnet, dass** die Bereiche der ersten Anlagefläche (42) und der zweiten Anlagefläche (44), welche sich gegenüberliegen, so gegen die Längsachse (LA) geneigt sind, dass ihr Abstand in Richtung zum distalen Ende hin abnimmt.

6. Endoskop nach einem der obigen Ansprüche, **dadurch gekennzeichnet, dass** der Innenrohrkopf (32) in Draufsicht auf das distale Ende (16) symmetrisch zu einer durch das Verbindungselement (38) verlaufenden Symmetrieachse ist.

7. Endoskop nach einem der obigen Ansprüche, **dadurch gekennzeichnet, dass** die beiden Anlageflächen (42, 44) zusammen mit dem Verbindungselement (38) in Draufsicht auf das distale Ende einen oder mehrere seitlich offene Aufnahmebereiche für die Lichtleiter bilden.

8. Endoskop nach einem der obigen Ansprüche, **dadurch gekennzeichnet, dass** das Innenrohr (28) und der Innenrohrkopf (32) zweiteilig ausgestaltet sind, wobei vorzugsweise das Innenrohr (28) und der Innenrohrkopf (32) durch Schweißen oder Löten miteinander verbunden sind.

9. Endoskop nach einem der obigen Ansprüche, **dadurch gekennzeichnet, dass** ein durch den ersten und zweiten Anlageabschnitt (34, 36) begrenzter Raum in Richtung zum distalen Ende hin kleiner wird.

10. Verfahren zum Fixieren wenigstens eines Bündels von Lichtleitern (31) in einem Schaft (14) eines Endoskops (10), umfassend die Schritte:
- Bereitstellen eines Mantelrohrs (26), welches sich entlang einer Längsachse (LA) erstreckt,
- Bereitstellen eines Innenrohrs (28), wobei das Innenrohr (28) an dem distalen Ende (16) einen Innenrohrkopf (32) mit einem ersten Anlageabschnitt (34) und einem zweiten Anlageabschnitt (36), der mit dem ersten Anlageabschnitt (34) durch ein Verbindungselement (38) verbunden ist, umfasst, wobei der erste Anlageabschnitt (34) eine erste Anlagefläche (42) und der zweite Anlageabschnitt (36) eine zweite Anlagefläche (44), welche der ersten Anlagefläche (42) wenigstens bereichsweise gegenüberliegt, aufweist, wobei ein Bereich der ersten Anlagefläche (42) und/oder ein Bereich der zweiten Anlagefläche (44) nicht-parallel zu der Längsachse (LA) verlaufen, und wobei das Verbindungselement (38) als Steg ausgebildet ist, und wobei der erste Anlageabschnitt (34), das Verbindungselement (38) und der zweite Anlageabschnitt (36) in Draufsicht auf das distale Ende (16) eine Doppel-T-Form bilden
- Ausrichten des Bündels von Lichtleitern (31) aus der Längsachse (LA) an dem distalen Ende (16) durch Anlegen des Bündels von Lichtleitern (31) an der ersten Anlagefläche (42) und der zweiten Anlagefläche (44), wobei das Bündel von Lichtleitern (31) an dem distalen Ende (16) durch das Verbindungselement (38) in mindestens zwei Teilbündel getrennt ist,
- Einführen des Bündels von Lichtleitern (31) und des Innenrohrs (28) in das Mantelrohr (26),
- Fixieren des Bündels von Lichtleiters (31) an dem Innenrohrkopf (32) und/oder dem Mantelrohr (26).

11. Verfahren nach Anspruch 10, **dadurch gekennzeichnet, dass** der Innenrohrkopf (32) einen Vorsprung (48), der in Richtung der Längsachse (LA) sowie senkrecht zu der Längsachse (LA) vorsteht, umfasst, wobei das Einführen des Bündels von Lichtleitern (31) und des Innenrohrs (28) in das Mantelrohr (26) derart erfolgt, dass der Vorsprung (48) in einer Ausnehmung (50) an dem distalen Ende (16) des Mantelrohrs (26) angeordnet wird.

12. Verfahren nach Anspruch 11, **dadurch gekennzeichnet, dass** das distale Ende (16) derart beschliffen wird, dass der Vorsprung (48) in seiner Ausdehnung in Richtung der Längsachse (LA) entfernt ist.

## Claims

1. Endoscope with a main unit (12) and a shaft (14), connected to the main unit (12), which extends along a longitudinal axis (LA),
- wherein the shaft (14) comprises a jacket tube (26), an inner tube (28) arranged in the jacket tube (26) for receiving an objective lens (18), and a bundle of fibre-optic light guides (31) which run along the longitudinal axis (LA) in the shaft (14) between the jacket tube (26) and the inner tube (28),
- wherein at a distal end (16) facing away from the main unit (12) the inner tube (28) comprises an inner tube head (32), which comprises a first bearing section (34) and a second bearing section (36), which is connected to the first bearing section (34) by a connecting element (38),
- wherein the first bearing section (34) comprises a first bearing surface (42), against which the bundle of fibre-optic light guides (31) abuts, and the second bearing section (36) comprises a second bearing surface (44), against which the bundle of fibre-optic light guides (31) abuts and which lies opposite the first bearing surface (42) at least in areas,
- wherein, to align the bundle of fibre-optic light guides (31) off the longitudinal axis (LA) at the distal end (16), an area of the first bearing surface (42) and/or an area of the second bearing surface (44) run non-parallel to the longitudinal axis (LA),
wherein the connecting element (38) is formed as a web,
wherein the bundle of fibre-optic light guides (31) is separated by the connecting element (38) into at least two partial bundles at the distal end (16) and the first bearing section (34), the connecting element (38) and the second bearing section (36), in top view onto the distal end (16), form a double T shape.

2. Endoscope according to claim 1, **characterized in that** the first bearing section (34) and/or the second bearing section (36) abut against an inner surface of the jacket tube (26).

3. Endoscope according to claim 1 or 2, **characterized by** an objective lens (18) which has an optical axis (OA) which forms an angle (α) with the longitudinal axis (LA) which is equal to an angle which the longitudinal axis (LA) forms with the area of the first bearing surface (42) and/or the area of the second bearing surface (44).

4. Endoscope according to any of the above claims, **characterized in that** partial areas (46) of the first bearing surface (42) and/or of the second bearing surface (44) are inclined to different extents relative to the longitudinal axis (LA), wherein the angle of inclination of the partial areas (46) preferably increases along a circumferential direction of the shaft (4).

5. Endoscope according to any of the above claims, **characterized in that** the areas of the first bearing surface (42) and of the second bearing surface (44) which lie opposite each other are inclined relative to the longitudinal axis (LA) such that the distance between them decreases in the direction towards the distal end.

6. Endoscope according to any of the above claims, **characterized in that** the inner tube head (32), in top view onto the distal end (16), is symmetrical relative to an axis of symmetry running through the connecting element (38).

7. Endoscope according to any of the above claims, wherein both bearing surfaces (42, 44), together with the connecting element (38), in top view onto the distal end, form one or more laterally open receiver areas for the fibre-optic light guides.

8. Endoscope according to any of the above claims, **characterized in that** the inner tube (28) and the inner tube head (32) are designed in two pieces, wherein preferably the inner tube (28) and the inner tube head (32) are connected to each other by welding or soldering.

9. Endoscope according to any of the above claims, **characterized in that** a space delimited by the first and second bearing sections (34, 36) becomes smaller in the direction towards the distal end.

10. Method of securing at least one bundle of fibre-optic light guides (31) in a shaft (14) of an endoscope (10), comprising the steps:
- providing a jacket tube (26) which extends along a longitudinal axis (LA),
- providing an inner tube (28), wherein at the distal end (16) the inner tube (28) comprises an inner tube head (32) with a first bearing section (34) and a second bearing section (36), which is connected to the first bearing section (34) by a connecting element (38), wherein the first bearing section (34) comprises a first bearing surface (42) and the second bearing section (36) comprises a second bearing surface (44), which lies opposite the first bearing surface (42) at least in areas, wherein an area of the first bearing surface (42) and/or an area of the second bearing surface (44) run non-parallel to the longitudinal axis (LA), and wherein the connecting element (38) is formed as a web, and wherein the first bearing section (34), the connecting element (38) and the second bearing section (36), in top view onto the distal end (16), form a double T shape
- aligning the bundle of fibre-optic light guides (31) off the longitudinal axis (LA) at the distal end (16) by placing the bundle of fibre-optic light guides (31) against the first bearing surface (42) and the second bearing surface (44), wherein the bundle of fibre-optic light guides (31) is separated by the connecting element (38) into at least two partial bundles at the distal end (16),
- inserting the bundle of fibre-optic light guides (31) and the inner tube (28) into the jacket tube (26),
- securing the bundle of fibre-optic light guides (31) on the inner tube head (32) and/or the jacket tube (26).

11. Method according to claim 10, **characterized in that** the inner tube head (32) comprises a protrusion (48) which protrudes in the direction of the longitudinal axis (LA) as well as perpendicularly to the longitudinal axis (LA), wherein the insertion of the bundle of fibre-optic light guides (31) and the inner tube (28) into the jacket tube (26) is effected in such a way that the protrusion (48) is arranged in a recess (50) at the distal end (16) of the jacket tube (26).

12. Method according to claim 11, **characterized in that** the distal end (16) is ground in such a way that the extent of the protrusion (48) is removed in the direction of the longitudinal axis (LA).

## Revendications

1. Endoscope comprenant une partie principale (12) et une tige (14) reliée à la partie principale (12) et qui s'étend le long d'un axe longitudinal (LA),
- la tige (14) comportant un tube d'enveloppe (26), un tube interne (28) disposé dans le tube d'enveloppe (26) et servant à accueillir un objectif (18) et un faisceau de fibres optiques (31) qui s'étendent dans la tige (14) le long de l'axe longitudinal (LA) entre le tube d'enveloppe (26) et le tube interne (28),
- le tube interne (28) possédant une tête de tube interne (32) au niveau d'une extrémité distale (16) à l'opposé de la partie principale (12), laquelle comporte une première portion d'application (34) et une deuxième portion d'application (36) qui est reliée à la première portion d'application (34) par un élément de liaison (38),
- la première portion d'application (34) possédant une première surface d'application (42) contre laquelle repose le faisceau de fibres optiques (31) et la deuxième portion d'application (36) possédant une deuxième surface d'application (44) contre laquelle repose le faisceau de fibres optiques (31) et qui se trouve au moins dans certaines zones à l'opposé de la première surface d'application (42),
- une zone de la première surface d'application (42) et/ou une zone de la deuxième surface d'application (44) s'étendant non parallèlement à l'axe longitudinal (LA) en vue d'orienter le faisceau de fibres optiques (31) à l'écart de l'axe longitudinal (LA) au niveau de l'extrémité distale (16),
l'élément de liaison (38) étant réalisé sous la forme d'une nervure,
le faisceau de fibres optiques (31) étant divisé par l'élément de liaison (38) en au moins deux faisceaux partiels au niveau de l'extrémité distale (16) et la première portion d'application (34), l'élément de liaison (38) et la deuxième portion d'application (36), vus de dessus sur l'extrémité distale (16), présentant la forme d'un double T.

2. Endoscope selon la revendication 1, **caractérisé en ce que** la première portion d'application (34) et/ou la deuxième portion d'application (36) reposent contre une surface interne du tube d'enveloppe (26).

3. Endoscope selon la revendication 1 ou 2, **caractérisé par** un objectif (18) qui possède un axe optique (OA), lequel forme avec l'axe longitudinal (LA) un angle (α) qui est le même angle qui forme l'axe longitudinal (LA) avec la zone de la première surface d'application (42) et/ou la zone de la deuxième surface d'application (44).

4. Endoscope selon l'une des revendications précédentes, **caractérisé en ce que** des zones partielles (46) de la première surface d'application (42) et/ou de la deuxième surface d'application (44) sont inclinées avec des amplitudes différentes vers l'axe longitudinal (LA), l'angle d'inclinaison des zones partielles (46) augmentant de préférence le long d'une direction circonférentielle de la tige (4).

5. Endoscope selon l'une des revendications précédentes, **caractérisé en ce que** les zones de la première surface d'application (42) et de la deuxième surface d'application (44) qui sont mutuellement opposées sont inclinées vers l'axe longitudinal (LA) de telle sorte que leur écart diminue en direction de l'extrémité distale.

6. Endoscope selon l'une des revendications précédentes, **caractérisé en ce que** la tête de tube interne (32), vue de dessus sur l'extrémité distale (16), est symétrique par rapport à un axe de symétrie qui passe à travers l'élément de liaison (38).

7. Endoscope selon l'une des revendications précédentes, **caractérisé en ce que** les deux surfaces d'application (42, 44), conjointement avec l'élément de liaison (38), forment en vue de dessus sur l'extrémité distale une ou plusieurs zones d'accueil ouvertes latéralement pour les fibres optiques.

8. Endoscope selon l'une des revendications précédentes, **caractérisé en ce que** le tube interne (28) et la tête de tube interne (32) sont configurés en deux parties, le tube interne (28) et la tête de tube interne (32) étant de préférence reliés ensemble par soudage ou brasage.

9. Endoscope selon l'une des revendications précédentes, **caractérisé en ce qu'**un espace délimité par la première et la deuxième portion d'application (34, 36) devient plus petit en direction de l'extrémité distale.

10. Procédé pour fixer au moins un faisceau de fibres optiques (31) dans une tige (14) d'un endoscope (10), comprenant les étapes suivantes :
- fourniture d'un tube d'enveloppe (26) qui s'étend le long d'un axe longitudinal (LA),
- fourniture d'un tube interne (28), le tube interne (28) comportant au niveau de l'extrémité distale (16) une tête de tube interne (32) comprenant une première portion d'application (34) et une deuxième portion d'application (36), qui est reliée à la première portion d'application (34) par un élément de liaison (38), la première portion d'application (34) possédant une première surface d'application (42) et la deuxième portion d'application (36) une deuxième surface d'application (44), laquelle se trouve au moins dans certaines zones à l'opposé de la première surface d'application (42), une zone de la première surface d'application (42) et/ou une zone de la deuxième surface d'application (44) s'étendant non parallèlement à l'axe longitudinal (LA) et l'élément de liaison (38) étant réalisé sous la forme d'une nervure, et la première portion d'application (34), l'élément de liaison (38) et la deuxième portion d'application (36), vus de dessus sur l'extrémité distale (16), présentant la forme d'un double T
- orientation du faisceau de fibres optiques (31) à l'écart de l'axe longitudinal (LA) au niveau de l'extrémité distale (16) par application du faisceau de fibres optiques (31) contre la première surface d'application (42) et la deuxième surface d'application (44), le faisceau de fibres optiques (31) étant divisé par l'élément de liaison (38) en au moins deux faisceaux partiels au niveau de l'extrémité distale (16),
- introduction du faisceau de fibres optiques (31) et du tube interne (28) dans le tube d'enveloppe (26),
- fixation du faisceau de fibres optiques (31) au niveau de la tête de tube interne (32) et/ou du tube d'enveloppe (26).

11. Procédé selon la revendication 10, **caractérisé en ce que** la tête de tube interne (32) comporte une partie saillante (48) qui se dresse dans la direction de l'axe longitudinal (LA) ainsi que perpendiculairement à l'axe longitudinal (LA), l'introduction du faisceau de fibres optiques (31) et du tube interne (28) dans le tube d'enveloppe (26) s'effectuant de telle sorte que la partie saillante (48) est disposée dans un évidement (50) au niveau de l'extrémité distale (16) du tube d'enveloppe (26) .

12. Procédé selon la revendication 11, **caractérisé en ce que** l'extrémité distale (16) est poncée de telle sorte que la partie saillante (48) est retirée dans son expansion dans la direction de l'axe longitudinal (LA).
